# EUROPEAN PATENT APPLICATION

(11) **EP 4 624 046 A1**
(43) Date of publication of application: **01.10.2025**
(21) Application number: 24180203.2
(22) Date of filing: 05.06.2024
(51) Int. Cl.: B01L 3/00

(54) **DEVICE FOR TESTING ANALYTE IN LIQUID SAMPLE**

(30) Priority: 27.03.2024 CN 202410360338
(71) Applicant: Zhejiang Orient Gene Biotech Co., Ltd, Anji Huzhou Zhejiang 313300 (CN)
(72) Inventor: Zhang, Ying, Huzhou, Zhejiang, 313300 (CN); Fang, Jianqiu, Huzhou, Zhejiang, 313300 (CN)
(74) Representative: Petraz, Gilberto Luigi

(57) **Abstract**

The invention discloses a testing device. The testing device includes: a chamber for receiving a liquid sample; a cover, where the cover is configured to seal the chamber, where the cover further includes a carrier with a groove, the carrier is configured to receive and hold a test strip, the cover further includes a hole that is connected to the groove, and the cover is provided with a label and a connection line between the label and the hole is shortest; and when testing is performed, the label is configured to indicate an inclination direction, such that the liquid sample contacts with testing areas of the test strip in the groove. The testing device can be used for quickly performing testing and is easy to operate, and test results thereof are more accurate.

## Description

### CROSS-REFERENCE TO RELATED APPLICATIONS

This application claims priority to the Chinese Patent Application, Application No. CN2024103603381, filed on March 27, 2024, and all disclosures of the Applications, including but not limited to the specification, abstract, claims and accompanying drawings of this application are hereby made a part of this application.

### BACKGROUND OF THE INVENTION

### Field of the Invention

The invention belongs to the field of in vitro diagnosis and is used for testing an analyte in a liquid sample, and in particular, to a device for testing some analytes in a urine sample and the properties of urine.

### Description of the Related Art

In the detection field, a testing device is a common medical testing instrument, which is mostly used to detect test samples in stored liquids, such as urine, blood, sewage, semi-solid substances (which refers to liquid samples converted by any suitable method), and the like. A common testing tool for testing a liquid sample is a reagent strip. Usually, there are two ways for the reagent strip to obtain the liquid sample. One way is to put the reagent strip directly into the liquid sample, and in this case, the bottom of the reagent strip is in contact with the liquid sample, such that the reagent strip can obtain the liquid sample; the other way is a dropping method, that is, the liquid sample is absorbed by a dropper and other tools and then dropped to the reagent strip, such that the reagent strip can obtain the liquid sample. In these two common ways, the reagent strip is in local contact with the liquid sample; usually, it can only be tested for one item. When the test item is changed, it is necessary to change a different reagent strip.

In addition, when there are multiple test items on a test strip, especially when biochemical tests are used, it is desired that each testing area of the test strip will contact with the liquid sample, such that the liquid sample can have a same reaction time in each testing area and the test results within the same time can be judged. The manual operation of the test is unsafe and unsanitary if used.

It is necessary to further improve an existing conventional device, so a convenient, hygienic and safe device is used for testing, and especially a chemical method is used for testing the analyte in the urine.

### BRIEF SUMMARY OF THE INVENTION

In order to overcome the disadvantages of the prior art, a device for testing an analyte in a liquid sample is provided. The device includes a chamber for receiving the liquid sample, where the chamber includes an opening and transparent side walls enclosing the chamber.

In some embodiments, the chamber includes the opening and the body of the chamber, and the chamber is enclosed by the transparent side walls. In some embodiments, the chamber includes an edge with an opening, and the edge is provided with a screw thread that is meshed with a screw thread of a cover, such that the cover seals the opening of the chamber. In some embodiments, an opaque label paper covers a bottom of the chamber, and carries a maximum liquid level label and a minimum liquid level label. In some embodiments, the label paper will form a window area where the liquid sample is observable, instead of completely sealing the transparent side walls of the chamber. In some embodiments, the window area is conveniently provided with a color block with a standard colorimetric card, and the color block is used for comparing with the color of the testing areas of the test strip, so as to judge whether test results of the testing areas are positive or negative. In some embodiments, a protrusion is provided near the opening of the chamber, and is to limit the rotation of the cover, such that the cover is located in a fixed position of the chamber. When the cover includes a carrier, the carrier is inserted into the chamber, thus limiting the specific position of the carrier in the chamber.

In some embodiments, the invention further includes a cover, where the cover is configured to seal the opening of the chamber. In some embodiments, the testing device includes the carrier with the groove; and the carrier is configured to receive and hold the test strip. In some embodiments, the cover includes a hole, and the hole is located at one end of the groove and connected to the groove, and the hole is configured to allow a testing element to pass through the hole and enter the groove of the carrier. In some embodiments, the groove further includes a tubular structure, and the tubular structure is used for holding the test strip in the groove without being detached. In some embodiments, the cover includes a label for chamber inclination, and a distance between the label and the hole is shortest relative to a distance between the label and other positions. In some embodiments, the cover further includes a sealing plug for sealing the hole, the sealing plug is also provided with the label, and the label is also used to indicate an inclination direction of the chamber. In some embodiments, the groove is enclosed by a bottom surface and two side surfaces, the groove is provided with the opening, and the opening faces side walls of a collection chamber. In some embodiments, the groove includes one or more holes, and the holes are configured to allow a liquid to pass through and flow into the groove so as to contact with the testing areas of the test strip. Of course, after the liquid sample contacts with the testing areas, the liquid sample can flow out of the groove through these holes. In some embodiments, the groove has a tubular structure at one end close to the cover, and the tubular structure is configured to allow the test strip to be located in the groove instead of departing from the groove.

In some embodiments, the invention further provides a test strip, where the test strip is used for testing the presence or absence or content of an analyte in a liquid sample. In some embodiments, the test strip includes one or more testing areas, and the testing areas are used for testing the presence or absence or quantity of the analyte in the liquid sample. In some embodiments, the test strip includes a non-absorbent support sheet on which one or more testing areas are provided, and the testing areas include an absorbent material. In some embodiments, the testing areas of the test strip include chemical substances, and the chemical substances are capable to react directly or indirectly with the analyte in the liquid sample to produce colored substances. In some embodiments, the analyte may be pH in urine or one or more of the analytes in the urine, for example, hemameba or leukocyte, erythrocyte, urobilinogen, urine vitamin c, urine crystal, urine specific gravity, urine albumin, urine ketone bodies, urine colony count, urine pH, and nitrous acid. In some embodiments, the test strip is provided with two same testing areas that include same chemical substances and are used for testing same analytes, and the testing areas are respectively distributed at both ends of the test strip. In some embodiments, the test strip includes two groups of testing areas, where each group of testing areas includes two or more testing areas for testing different analytes, and the two groups of testing areas include same testing areas for testing same analytes.

In addition, the invention provides a method for testing an analyte in a liquid sample, and the method includes: providing a testing device, where the testing device includes: a chamber for receiving the liquid sample, where the chamber includes an opening and transparent side walls enclosing the chamber; a cover, where the cover is configured to seal the opening of the chamber, the cover further includes a carrier with a groove, the carrier is configured to receive and hold a test strip, the cover further includes a hole, the hole is located at one end of the groove and connected to the groove, and the hole is configured to allow a testing element to pass through the hole and enter the groove of the carrier and sealed by a sealing plug; and the test strip, where the test strip includes one or more testing areas, and the testing areas are used for testing the presence or absence or quantity of the analyte in the liquid sample; the cover is provided with a label and a connection line between the label and the hole is shortest; when testing is performed, the label is configured to indicate an inclination direction, such that the liquid sample contacts with the testing areas of the test strip in the groove; the liquid sample is collected by the chamber and located at the bottom of the chamber, where the liquid sample is a urine sample; the opening of the chamber is sealed by the cover, such that the carrier is inserted into the chamber;

the sealing plug is opened, the test strip is inserted into the groove through the hole in the cover, and then the hole is sealed by the sealing plug; and the chamber is allowed to be inclined towards a direction indicated by the label on the cover for 1-10 seconds and stand again.

In some embodiments, when the test strip is inserted into the groove of the carrier, the testing areas of the test strip are allowed to approach the hole of the cover; in addition, the test strip includes a non-absorbent support sheet, and the testing areas are located on the support sheet and include an absorbent material.

### BRIEF DESCRIPTION OF THE DRAWINGS

FIG. 1 is a schematic diagram showing a three-dimensional structure of a detection structure (excluding a test strip) according to a specific embodiment of the invention.
FIG. 2 is a schematic diagram showing a three-dimensional structure of a chamber according to the invention, where the chamber includes a carrier and a testing element on the carrier, and a positive color block and a negative color block on the chamber have been provided for interpreting results.
FIG. 3 is a structural schematic diagram of a cover and a combination of a carrier and the cover according to a specific embodiment of the invention.
FIG. 4 is a schematic diagram showing a three-dimensional structure of a cover according to the invention, where the cover carries a chamber inclination direction label (when the cover and the chamber are combined or when the cover and the chamber are combined for testing).
FIG. 5 is a schematic diagram showing a three-dimensional structure of a sealing element according to the invention, where the sealing element also carries a schematic diagram of an inclination direction.
FIG. 6 is a structural schematic diagram of a test strip according to a specific embodiment of the invention.
FIG. 7 is a structural schematic diagram of a test strip according to a specific embodiment of the invention.
FIG. 8 is a structural schematic diagram of a test strip according to a specific embodiment of the invention.
FIG. 9 shows a specific embodiment according to the invention, where during testing, a chamber contains a liquid, a carrier is located in the chamber, and a test strip is also located on the carrier.
FIG. 10 is a structural schematic diagram showing an inclination along a direction as indicated by an arrow on a cover.
FIG. 11A is an illustrative diagram of a principle according to a specific embodiment of the invention.
FIG. 11B is a structural schematic diagram of a principle of an inclination along a direction as indicated by a hole and an arrow.
FIG. 11 C is a structural schematic diagram of a principle of an inclination along a direction indicated by a hole and a dot.

### DETAILED DESCRIPTION OF THE INVENTION

### Detection

Detection means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein or a polymer. In addition, detection means that the amount of a substance or material is tested. Further, assay also means immunoassay, chemical assay, enzyme assay, and the like.

### Samples

Samples that can be tested by the testing device of the invention include biological liquids (for example, case liquids or clinical samples). Liquid samples or liquid specimens may be derived from solid or semi-solid samples, including feces, biological tissues and food samples. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine; and preferably, the biological sample is saliva, sputum, nasal secretion, or the like. Food samples include food processing substances, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other waste water.

In some embodiments, the sample of the invention may be a urine sample of human or mammal. Therefore, the device of the invention can be used for testing whether a liquid medicine is infected, or specific analytes in urine or the content of the analytes.

### Downstream and upstream

Downstream or upstream is divided according to a flow direction of a liquid. Generally, a liquid flows from an upstream area to a downstream area. The downstream area receives the liquid from the upstream area, and a liquid also may flow to the downstream area along the upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and in this case, downstream or upstream is divided according to a flow direction of the liquid. For example, in the collection device of the invention, in some preferred embodiments, the first chamber serves as a chamber for collecting a liquid sample, while the second chamber is in fluid communication with the first chamber, and the liquid flowing into the first chamber flows into the second chamber. The first chamber can be called upstream while the second chamber can be called downstream. Of course, such flow is natural flow of liquid under the action of gravity. Optionally, such natural flow is the flow of the liquid from the first chamber to the second chamber.

### Gas communication or liquid communication

Gas communication or liquid communication means that liquid or gas can flow from one place to another. In the flow process, the liquid or gas may pass through some physical structures that play a guiding role. The "pass through some physical structures" here means that the liquid passes through the surface of these physical structures or the internal space of these physical structures and flows to another place passively or actively, where the passive flow is usually caused by external forces, such as the flow under the capillary action. The flow here may also be a flow due to self-action (gravity or pressure) of the liquid or gas, and also may be a passive flow.

### Detachable combination

A detachable combination means that two components are connected in several different states or positional relationships. For example, with two components being physical components, they can be separated at the beginning and then connected or combined in an appropriate first case, and separated in an appropriate second case. Physically, such separation is spatial separation without contact. Alternatively, the two components are combined at the beginning, and can be physically separated from each other when appropriate. In short, combination or separation of two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component.

### Testing element

The testing element can be a lateral flow test strip that can detect a variety of analytes. Of course, other appropriate testing elements also can be used in the invention, and an element that can be used to detect whether a sample or a specimen contains an interested analyte may be called as the testing element. Such testing can be based on any technical principles, such as immunology, chemistry, electricity, optics, and physics. Various testing elements can be combined for use in the invention. One form of the testing elements is a test strip.

Test strips used in the invention may be commonly referred as lateral flow test strips. The specific structure and testing principle of the test strips are well known to a person skilled in the art in the prior art. A common test strip includes a sample collection area, a label area, a testing area and a water absorption area. The sample collection area includes a sample receiving pad, the label area includes a label pad, and the water absorption area may include a water absorbent pad. The testing area includes necessary chemical substances for detecting the presence or absence of the analyte, such as immunoreagents or enzyme chemical reagents. The nitrocellulose membrane test strip is commonly used, that is, the testing area includes a nitrocellulose membrane, and a specific binding molecule is immobilized on the nitrocellulose membrane to display the test result; and other test strips such as cellulose acetate membrane or nylon membrane test strips may also be used. Of course, in the downstream of the testing area, there may also be a test result control area. Generally, the control area and the testing area in the form of horizontal lines, namely, a test line or a control line. Such test strips are conventional. Of course, they may also be other types of test strips for detection under the capillary action. In addition, there are dry chemical reagent components on common test strips, for example, an immobilized antibody or other reagents. When the test strip contacts a liquid, the liquid flows along the test strip under the capillary action, and the dry reagent components are dissolved in the liquid and treated in a next area, and the dry reagents react in the area for necessary detection. The liquid flow mainly relies on the capillary action.

An appropriate testing element according to the invention can be used to test any analyte. Preferably, the testing device of the invention is used to detect small drug molecules in saliva and urine. Of course, any samples of the above forms may be collected by the sample collector of the invention, regardless of being solid or liquid at the beginning, provided that these liquids or liquid samples flow to the collection chamber and the test strip is inserted from the cover of the sample collector for testing.

Therefore, in some embodiments, the test strip of the invention is made of a non-absorbent material as a supporting structure; there is an absorbent testing area on the non-absorbent material, and the absorbent testing area consists of the absorbent material; chemical substances are pretreated on the testing area and can react with the analyte to cause color change, thereby judging the presence or absence of the analyte or the form of the sample. Such reaction can be that the analyte directly reacts with a colored substrate to produce a new colored substance or the analyte reacts with some chemical substances to produce a new substance, and the new substance reacts with the colored substrate to produce a new colored substance. These substances may be selected from original colored substances produced in chemical reaction. These colored substances are precipitated and attached to the absorbent material on the testing area, such that the absorbent material can exhibit a specific color, and then the test results can be judged through comparison of the specific color with a standard colorimetric card.

Urine is taken as an example, and the pH value of the urine and the analyte in the urine can be tested, for example, hemameba or leukocyte, erythrocyte, urobilinogen, urine vitamin c, urine crystal, urine specific gravity, urine albumin, urine ketone bodies, urine colony count, urine pH, and nitrous acid. In some embodiments, any two or more of the above detection indexes can be selected for detection, so one or more absorbent materials, such as filter paper blocks, filter paper sheets and absorbent sheets, are arranged on the non-absorbent material as the supporting structure. Substantially, each absorbent material is considered as one testing area that includes an absorbent pad or an absorbent sheet, and an analyte is tested on each testing area. When it is desired that two or more kinds of analytes are tested on the testing area, two or more absorbent sheets are pasted on the non-absorbent material as the supporting structure, and the chemical substances are treated on the absorbent sheets and can react with two or more kinds of analytes in the sample and produce colors. These colored substances are precipitated on the corresponding absorbent blocks or absorbent sheets, and then the colors are compared by the standard colorimetric card to judge whether the corresponding analyte is positive or negative.

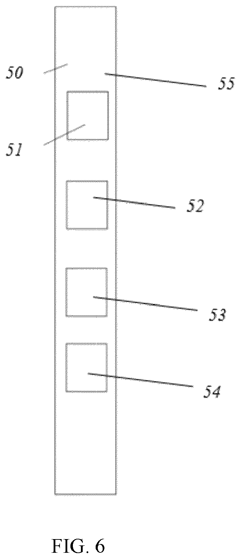

In some embodiments, for example, as shown in FIG. 6 to FIG. 8, there are four testing areas 51, 52, 53, 54 on one test strip; the analytes tested in these testing areas are different; and the four testing areas are all arranged on the non-absorbent sheet 55. These testing areas are arranged in sequence along the longitudinal axis of the test strip, and the analyte corresponding to each testing area can be printed or labeled on the support sheet 55. Of course, the analyte and the color blocks corresponding to the negative or positive of the analyte can also be labeled on the provided standard colorimetric card in a corresponding sequence on the test strip. Generally, the non-absorbent support sheet can be hard paper, and a plastic or metal sheet, while the absorbent testing area consists of the filter paper; the chemical substances are pretreated on the filter paper and dried to make filter paper blocks for testing; or these filter paper blocks are dried by a long strip of filter paper treated with the chemical substances and then pasted on the non-absorbent support sheet 55.

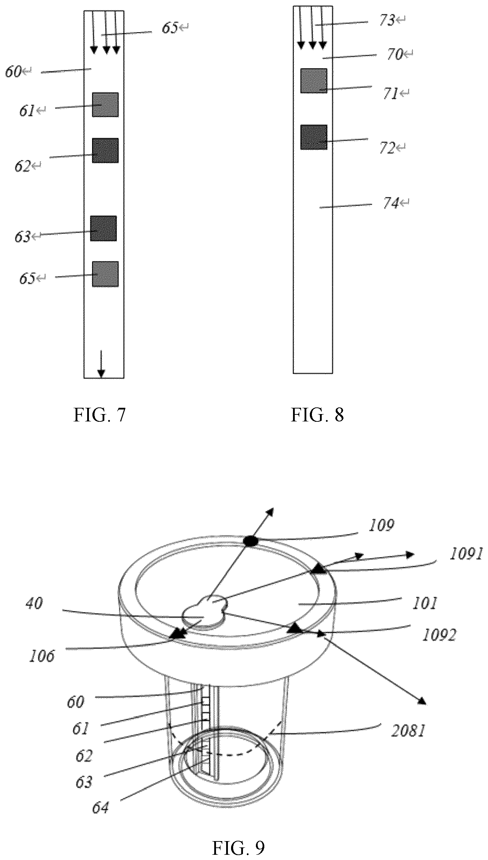

In some embodiments, as shown in FIG. 7, the test strip includes four testing areas 61, 62, 63, 64, where the analytes tested in the testing area 61 and the testing area 63 are the same; and similarly, the analytes tested in the testing area 62 and the testing area 64 are the same. The testing areas are spaced apart on the test strip 60, where the testing area 61 and the testing area 62 are arranged in sequence and the testing area 63 and the testing area 64 are arranged in sequence. One of such advantages is that the test strip needs to be inserted into the liquid sample; and when there is a small amount of the liquid sample in the collection chamber, the testing areas 63, 64 below the test strip can be moistened to complete testing. In addition, when there is the sufficient amount of the urine sample to moisten the four testing areas, it is enough to compare the test results of one testing area with the colourimetric card. In addition, if an operator is not careful, no matter which direction the test strip is inserted into the carrier and one end of the test strip is immersed in the liquid sample, two testing areas are always kept dry before the test, such that testing can be successfully completed. The test strip also has the function, for example, in some cases, one of the four testing areas may be moistened in advance due to careless operation. For example, if the testing area 64 is moistened in advance by water, the testing area 62 can still be used for testing the analyte in urine, thereby ensuring that the test strip can complete the whole test without being wasted. For example, when one test strip 60 is pulled out from a box where the test strips are stored, one end 65 of the test strip indicated by an arrow is generally held by hand and sometimes may fall off from the hand to an experimental table carelessly; there may be the liquid such as water on the experimental table, so individual testing areas on the test strip 50 will be moistened in advance, for example, the testing area 61 will be moistened in advance by water, and if the testing area 61 moistened in advance is used to contact with the urine, the testing area 61 moistened in advance contains water, and the saturated water absorption capacity of one testing area is customized. The so-called saturated water absorption capacity is the maximum volume of water absorbed by the testing area started from a dry state; once the testing area reaches the maximum volume, it can no longer absorb the water. When the absorbent material in the testing area absorbs enough water to reach the saturated water absorption capacity or absorbs water (without reaching the saturated water absorption capacity), if the absorbent material contacts with real liquid samples such as urine samples, the testing area cannot absorb more urine or absorbs some urine, meaning that the water for in advance moistening the testing area has a dilution effect on the urine; thus, the concentration of the analyte in the real urine in the testing area 61 is reduced, causing false negative results and even failing to react with the analyte in the urine. Generally, the urine needs to immediately depart from the testing area after instantaneously contacting with the testing area, but the testing area cannot be immersed in the liquid sample for a long time. The so-called instantaneous or short-time contact is about 1-2 seconds, and 1-10 seconds, and then the urine is allowed to depart from the testing area, such that the chemical substances on the testing area react with potential analytes in the urine staying in the testing area to produce colored substances and stay in the testing area. In this case, if a backup testing area 63 remains dry, the dry testing area 63 can still be allowed to contact with the urine, so as to obtain real test results. In particular, when one test strip includes 5-10 or even more testing areas, one testing area should not be abandoned because it is moistened in advance; therefore, the test strip can be provided with two or more same testing areas as backup testing areas. The following will make detailed descriptions with reference to operations. Such arrangement can not only satisfy the operation of professionals, but also satisfy those home self-testers who have no operating experience to operate by themselves.

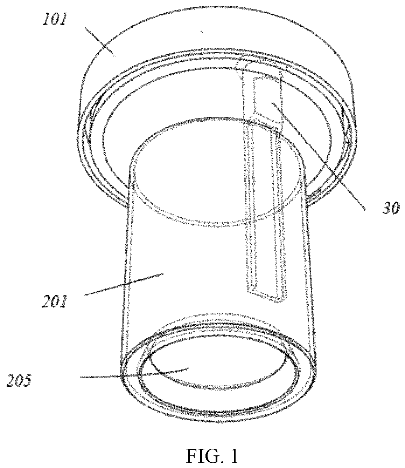

Such arrangement also has another advantage. For example, as shown in FIG. 7, the testing areas 61, 63, 62, 64 where same analytes are tested are arranged in sequence. For example, the analytes tested in the testing areas 61, 62 (such as hemameba (testing area 61) and nitrous acid (testing area 62)) are the same as the analytes tested in the testing areas 63, 64 (hemameba (testing area 63) and nitrous acid (testing area 64)). During testing, it is actually desired that the sample will depart from immediately after contacting the testing area, and the testing area cannot be immersed in the liquid sample all the time. Actually, two same testing areas of the testing areas are allowed to be located respectively at both ends of the test strip, or two testing areas and other two testing areas are respectively located at both ends of the test strip. When the device of the invention is used for testing, the test strip is allowed to be inserted into the chamber through an insertion mechanism and kept in the chamber all the time; however, the chamber 201 is used for receiving the liquid sample (as shown in FIG. 1 or FIG. 9), if the liquid sample in the chamber is excessive and exceeds a maximum line (MAX), for example, the level of the liquid exceeds the position of the testing area 64 on the test strip 60, the testing area 64 will always be immersed in the liquid during the test. With the testing area being immersed in the liquid, the colored substance will be dissolved or dispersed in the urine in case of the colored substances, such that the colored substances will not be accurately precipitated on the testing area 64, and the colored substances on the testing area will be reduced, resulting in inaccurate test results arising from false negative obtained through comparison of the colors of the colored substances with the external colorimetric card. However, in the invention, there is also a same testing area 62 located on the test strip 50, and the testing area 62 is located above the level of the sample, the liquid and the testing area 62 are allowed to immediately depart from urine after contacting with the urine, or the urine is allowed to immediately depart from the testing area 62 after contacting with the testing area, such that the analyte on the testing area 62 can be tested. Therefore, for the test strip as shown in FIG. 7, as long as the level of the urine is below the testing area 62, intended analytes still can be tested in the testing areas 61, 62. Therefore, testing can be completed by inserting the test strip as shown in FIG. 7 into the carrier and the chamber in any directions; for example, the test strip can be inserted into the chamber along a direction as indicated by a single arrow of FIG. 7 or can be inserted into the carrier along a direction opposite to a direction as indicated by three arrows. FIG. 8 shows one test strip. Two testing areas 71, 72 are provided on the non-absorbent support sheet 70, and these absorbent materials are located at one end of the test strip. When the test strip is inserted into the carrier and then the chamber 201 along the direction opposite to a direction as indicated by three arrows, the testing areas at the end of the test strip can contact with the liquid sample, but is always immersed in the liquid sample for a long time, which can result in false-negative test results.

However, a fact that the test strip itself is used to contact with the liquid in the chamber 201 to test the presence or absence of the analyte in the liquid sample is excluded. In some preferred embodiments, the testing element can also be provided on a test card with many grooves, where the testing element is located in the grooves, and the whole test card can be inserted into the chamber 201, and then the liquid sample in the chamber can be allowed to contact with multiple testing areas on the test strip to test the analyte. Of course, these test cards with test strips can be located in the chamber 201; then, when the collected liquid sample flows into the chamber 201, the liquid sample is allowed to contact with multiple testing areas on the test strips, thus completing the testing of the analyte in the liquid sample.

For example, in some embodiments, the liquid sample can be firstly collected in the chamber 201, and then the test strip or the test card with the test strip or the carrier is inserted into the first chamber for testing. In a preferred embodiment of the invention, the chamber 201 is used to receive or collect the liquid sample, and then the carrier 30 is inserted into the chamber 201, and then the test strip is placed on the carrier, such that the liquid in the chamber 201 contacts with the testing areas of the test strip, such as a plurality of testing areas. The testing areas are used to test the analyte in the liquid sample; and after the test, the test strip is kept in the chamber 201 and the carrier 30. In some embodiments, the carrier 30 has one groove that is provided with the test strip of the invention, for example test strips 50, 60, 70 (FIG. 6 - FIG. 8). If there are multiple grooves, each groove can be provided with one test strip, and each test strip has multiple testing areas. The groove is an open groove, such that the liquid can flow into the groove and contact with the testing area on the test strip, instead of chromatography. Because the support sheet supporting the testing area is not absorbent and the material on the testing area is absorbent, it is similar to allow the liquid sample to overflow all the test strips. In fact, some actions or ways are adopted to allow the liquid sample in the chamber 201 to actively move into the groove and overflow the groove, thereby wetting the testing areas and then quickly departing from the testing areas to prevent them from being soaked in the liquid sample for a long time.

### Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzodiazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The testing device of the invention may also be used to detect drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow detection in combination with the device of the invention. An appropriate testing element according to the invention can be used to detect any analyte. Preferably, the testing device of the invention is used to detect small drug molecules in saliva and urine. Preferably, the testing device can be used to detect small molecular substances such as viruses and bacteria in saliva, throat or nasal fluid. The testing device can also be used to detect white blood cells, red blood cells, urobilinogen, urine vitamin c, urine crystals, urine specific gravity, urine albumin, urine acetone bodies, urine colony count, urine pH and nitrous acid in the liquid samples. For example, the testing device can also be used to detect the levels of white blood cells, red blood cells, urobilinogen, urine vitamin c, urine crystals, urine specific gravity, urine albumin, urine acetone bodies, urine colony count, urine pH and nitrous acid in the urine sample.

The analyte in the urine can be tested by an immune method or a chemical method. Testing by the chemical method means that the absorbent material is treated with chemical substances. When the urine contains a specified amount of a specific analyte, a chemical reaction will occur on the absorbent material, and colored substances are produced and make the absorbent material colored. Through comparison of the colored substances and a standard colorimetric card, it is possible to know whether the analyte in the urine sample exists or how much it probably exists. Generally, the thick color indicates the high content of the analyte. For example, when the nitrous acid is tested, aromatic sulfadiazine is treated on the testing area and reacts with the nitrous acid to produce a diazo compound; the diazo compound reacts with 2,3,4-tetrahydrobenzo (h) quinoline -3-phenol to produce a pink color substance; and the pink color substance precipitates on the testing area, such that the testing area shows pink.

The white blood cells in urine contain esterase that can catalyze the hydrolysis of privatized pyrrole amino acid ester to release 3-hydroxy-5-phenylpyrrole. Then, the pyrrole reacts with diazonium salt to form purple. The reaction is used to test the content or number of the white blood cells in urine.

### Testing device

The testing device refers to a device for detecting the presence or absence of an analyte in a sample. The collection device refers to a device for collecting and storing liquid samples. The testing device can include a collection device, and the collection device can also include the testing device, or the collection device is separated from the testing device; during the test, the collection device and the detection device are combined to complete the test. Alternatively, the collection device and the detection device are of an integral structure, and therefore the collected liquid sample can be tested immediately to obtain the test result; in addition, the test sample can be separated from the collected sample, such that secondary test can be performed (if necessary). The testing device and the detection chamber here are interchangeable, and the collection device and the collection chamber are also interchangeable, but only their functions are interchanged depending on their different roles. For example, the collection device of the invention may not include the testing chamber or the first chamber, but the collection device may include the testing element or the carrier with the testing element; the collection device including the testing element may also be called the testing device, for example, the first chamber includes the testing element. Of course, the collection device can include a space where the testing element is provided, but does not necessarily include the testing element, and the testing element can be combined with the collection device at any suitable time to serve as the testing device. For example, the collection device may include a space for accommodating the testing element, such as the detection chamber; alternatively, the liquid collection chamber of the collection device has a suitable position where the testing element or the carrier including the testing element is provided.

### Collection chamber of testing device

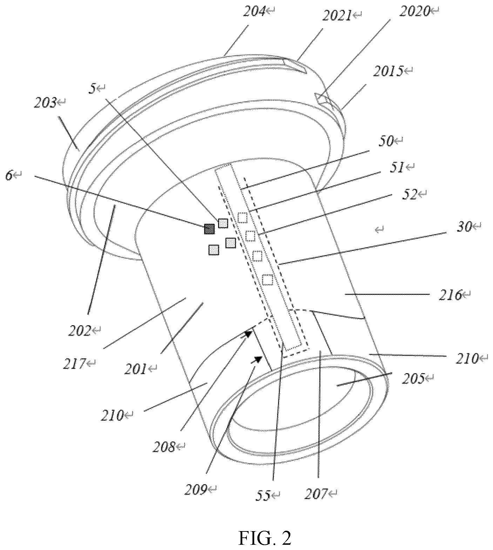

The testing device herein includes a liquid sample collection chamber, a cover, and a carrier for carrying test strips. In some embodiments, the collection chamber herein is a chamber for receiving a liquid sample and used for receiving the liquid samples, such as urine samples, as shown in FIG. 2. The collection herein can be that a test subj ect directly allows the liquid sample to flow into the chamber 201, or an operator adds the liquid sample into the chamber 201 through other tools, such as a pipette or other containers. Therefore, the chamber 201 is enclosed by a bottom 205 and side walls 216 connected with the bottom, and is transparent. In some embodiments, the chamber has an opening 204 for receiving the liquid sample. In some embodiments, the opening of the chamber 201 has an outer edge 203 with a screw thread 2015, and the chamber 201 is divided into two parts: the outer edge 203 of the opening 204 and the body 217 of the chamber 201, where the diameter of the outer edge 203 is larger than that of the body 217, such that the liquid sample can be conveniently received through the large outer edge with the opening 204, the small chamber 217 is used for storing liquids. Therefore, a connecting part 202 is provided between the small chamber 217 and the large outer edge 203, and is similar to a horn mouth. This has the advantage that the test subject can directly urinate into the chamber 201 through the large outer edge with the opening. In some embodiments, there is a surrounding label 210 on the outer wall of the chamber 201 near the bottom. The main function of the label is to record the information of the test subject, such as the time of collecting the sample, the name or number of the test subject. In some embodiments, the label is also provided with a maximum liquid level label (MAX) 209 (as indicated by an arrow) and a minimum liquid level label (MIN) 209 (as indicated by an arrow). This indicates the minimum liquid level and the maximum liquid level for the chamber 201, which is actually the amount of the liquid. This is a label indicating the maximum amount of the liquid sample and the minimum amount of the liquid sample. However, in some cases, if the test subject directly urinates into the chamber 201 during the test, the urine often exceeds the maximum liquid level label 208. In this case, setting of the testing area of the test strip (for example, the test strip as shown in FIG. 7 is used) can avoid some potential problems, which will be explained again with specific examples. In some embodiments, the outer walls of the bottom of the whole chamber are not surrounded by the label, but are reserved with a window area not covered by the label; the level or state of the liquid sample can be observed through the transparent window area, and this will be described in detail later. Generally, the label is opaque, so if the body 217 of the chamber is transparent, and the label 210 at the bottom actually makes a part of the chamber opaque.

In some embodiments, if a color change is made on the testing areas, the color needs to be compared with the standard color card to judge the test results and the whole chamber 201 needs to be close to the color card, which is somewhat inconvenient. Therefore, one way is that the standard color block can be directly printed on the transparent walls of the chamber 201, and another way is that the standard color block is printed on the surface of a self-adhesive sticker, the self-adhesive sticker is attached on the side wall of the chamber and close to the carrier 30; once the testing areas in the carrier 30 are tested, their color can be directly compared with the standard color card nearby them to judge whether the testing results are negative or positive. If the standard color card is fixed onto the surface of the chamber 201, as shown in FIG. 2, different chambers and carriers are located in the same position or substantially the same position in the chamber 201 by using some ways, which is more convenient for judging the results.

### Cover and carrier with the cover

In some specific embodiments of the invention, the testing device further includes one cover 10, where the cover is used for sealing the opening 204 of the chamber 201 and provided with a screw thread 103, and the screw thread 103 is meshed with the screw thread 2015 on the outer edge of the opening 204 of the chamber, thereby sealing the chamber. In some embodiments, the testing device further includes a carrier 30 for carrying a test strip, where the carrier is provided with a groove 301 for carrying the test strip (for example, the test strips as shown in FIG. 6 - FIG. 8). One function of the carrier is to allow the test strip to lean against or be held in the groove; another function thereof is to allow the test strip to be inserted from one end of the carrier into the chamber and the carrier plays a guiding role; still another function thereof is to allow the liquid to flow into the groove and contact with the testing areas of the test strip; and yet still another function thereof is to allow the test strip to be still held in the groove to avoid slipping after the liquid departs from the testing areas. In some embodiments, the carrier and the cover are of an integrated structure. The carrier 30 has two ends, and there is a groove 301 between one end 305 and the other end 308. The groove is enclosed by a bottom surface 309 and two sides 310, 311, while the other end 308 is directly connected with the cover. The groove 301 herein is actually open instead of a pipe, which is convenient for the liquid sample to quickly flow into the groove and contact with the testing areas of the test strip during the subsequent test. One hole 105 is provided in the cover, and the other end 308 of the groove is directly connected to the hole 105 in the cover, that is, when the test strip is inserted through the hole 105 in the cover, the test strip actually enters the groove and always extends forward. Therefore, the test strip can be inserted into the chamber 201 through the hole 105 in the cover, and the groove can be used as a specific position where the test strip is guided to be inserted into the chamber; in other words, when the test strip is inserted into the chamber 201 from the hole 105 and contacts with the urine, the test strip can only enter the chamber along the direction of the groove, but cannot be inserted into other positions. The groove actually plays the role of a specific limited position for the test strip to enter the chamber 201. In some embodiments, there is a cover plate 303 at one end of the groove near the cover, and the cover plate 303 covers a section of the groove and the opening of the groove to form a structure of a pipe 302. When the test strip is inserted into the groove through the structure of a pipe 302, it is not easy for the test strip to be separated from the groove in a subsequent operation. In some practical operations, in some cases, the test strip includes a non-absorbent support sheet, and one or more absorbent testing areas are provided on the support sheet. When the test strip is inserted into the chamber 201 through the hole 105 in the cover, there is the liquid sample in the chamber 201. When the test strip is inserted, it is not desired that the testing areas of the test strip will contact with the liquid sample, so it is desired that the testing areas will remain dry. After insertion, the liquid sample in the chamber 201 can be allowed to contact with the testing areas by inclining and shaking the chamber 201 or other shaking methods, such that the testing areas can absorb the liquid sample. Once the liquid sample contacts with the testing areas for 1-10 seconds, other liquid samples are allowed to depart from the testing areas. Similarly, the testing areas are allowed to be immersed in the liquid sample for a very short time (for example, for 1-10 seconds), the testing areas are allowed to depart from the liquid sample, chemical reagents on the testing areas react with the analyte in the liquid sample to produce colored substances, and the colored substances are deposited on the testing areas. In a process of inclining, shaking or swaying the chamber, the liquid will have a certain impact on the groove 30, so it is desired that the test strip will still be in the groove and will not be detached from the groove. Therefore, the structure 304 of the pipe is to avoid falling, such that the test strip is not detached from the groove 30, does not float in the liquid, or is not completely immersed in the liquid sample during operation, or it is not desired that some testing areas of the test strip will always be immersed in the liquid sample especially when there are many testing areas of the test strip. In this case, the function to limit the pipe 302 is more important. In some cases, especially when the length of some test strips is less than the length of the whole groove, or when the length of the test strips is the same as the length of the groove, the structure of the pipe 302 is particularly helpful for limiting detachment of the test strip from the groove, which can effectively prevent the test strips from falling off the groove and causing some testing areas to be immersed in the liquid sample for a long time in a specific operation process, especially when the liquid is allowed to actively contact with the testing areas of the test strips. In some embodiments, most of the grooves are open without a cover to expose the testing areas of the test strips, such that the color change or color shade on the testing areas is conveniently observed through some transparent side walls of the chamber 201 so as to judge the test results.

In some embodiments, the bottom 305 of the groove can also be provided with many small holes 306. The function of the small holes is that when the chamber 201 is swayed, shaken or inclined, the liquid can quickly flow into the groove and contact with the testing areas of the test strip in the groove, and the liquid sample can flow into the groove through the small holes, so that the liquid sample can contact with the testing areas as much as possible and depart from the testing areas in a short time. Of course, when it is desired that the liquid will depart from the groove, the liquid can quickly flow out of the groove through these small holes, and the testing areas are kept in a relatively independent position. The testing areas consists of an absorbent material, and necessary chemical reagents that react with the analyte are processed on the testing areas. These chemical reagents contact with the analyte in the liquid sample; and if there is the analyte on the testing areas, direct or indirect reaction is made, thus producing colored substances retained by the testing areas. If the testing areas are immersed in the liquid sample for a long time, the colored substances will be dissolved or diffused in the liquid sample to reduce the color of the testing areas and further reduce the concentration; the color change is not obvious, which may lead to false negative results. However, the colored substances diffused in the urine may be adsorbed or absorbed by another testing area, so the another testing area has color, which may lead to false positive results. In short, the testing areas should not be immersed in the liquid sample for a long time, and the liquid sample should depart from the testing areas as quickly as possible after momently contacting with the testing areas.

In some embodiments, shaking the liquid or inclining the chamber 201 is to allow the liquid sample to actively contact with the testing areas of the test strip, while the test strip is kept in a relatively fixed position. One of ways to determine that the liquid has contacted with the testing areas through observation and judgment made by naked eyes of an operator. When the chamber is held in the hand during operation, whether the urine has contacted with all the testing areas is observed through a part of the transparent side walls 216 of the chamber 201. If the urine has contacted with all the testing areas, the chamber is manually placed in an upright position to allow the liquid to depart from the testing areas. In some embodiments, in order to observe the structure of the testing area more conveniently, for example, when the liquid sample is located at the minimum liquid level label and if the there are many testing areas of the test strip, some testing areas (for example, a dashed testing area 55 in FIG. 2 is located between the minimum liquid level label and the maximum liquid level label) are blocked by the labels on the chamber. In this case, it is desired that the carrier will stay at the fixed position (for example, at the fixed position as shown in FIG. 2) of the chamber every time. The carrier stays in an area defined by the window area, such that the test results of all the testing areas can be observed. If the carrier 30 stays in a fixed area every time, the standard color card can also be provided near the area to facilitate the interpretation of the test results. For example, as shown in FIG. 2, the standard color block 5, 6 is provided near the area to facilitate the interpretation of the test results. One of solutions to allow the carrier 30 to be located at the fixed position of the chamber when different testing devices (for different individual liquid samples) are used for testing is that the cover and the opening of the chamber are sealed in a piston form. For example, the inside 102 of the cover is provided with one protrusion similar to a piston, and the protrusion is directly inserted in the opening of the chamber or clamped on the opening, such that the carrier 30 can be located at the fixed position of the chamber every time. Another solution is to design the screw thread of the cover and the screw thread of the chamber 201. The starting position of the screw thread of the cover and the starting position of the screw thread of the chamber can be designed. In the invention, the screw thread of the chamber has a circle of screw threads, the starting position 2021 and the ending position 2020 are located at a same longitudinal position, and the cover 10 also substantially corresponds to a circle of screw threads 103. The starting position (not shown) and the ending position 2020 are consistent with the opening of the chamber; no matter where the cover is meshed with the chamber, finally the screw threads of the chamber are used up still at a same position. Therefore, after the screw threads of the cover and chamber are used up, the cover and the chamber cannot rotate and are located at one same position. Here, when a plurality of testing devices are combined, the position where the cover seals the chamber is in the same or substantially the same position of each testing device, that is, the starting position 2021 and ending position 2020 of the screw threads 2015 on the outer edge 203 of the opening 204 of the chamber in FIG. 2, and the cover stays here because the screw threads are used up. Therefore, when the carrier is located in the chamber 201, the positions where different testing devices stay are substantially the same, for example, they all stay in the transparent area where the window area is located. This solves the problem that some testing areas are blocked by the label and fixedly provided with the standard color card, and all the testing areas of the test strip can be observed conveniently.

For the conventional product, although the screw thread 103 of the cover 10 is fitted with the screw thread 2015 of the chamber 201, the opening 204 is sealed by rotating the screw threads of the cover and chamber and the carrier 30 is driven to rotate through the rotation of the cover 10; and when the screw threads are used up, the cover cannot rotate again and the carrier will be fixed. In fact, the starting position where the screw threads of the cover and the screw threads of the chamber 201 are meshed is random, so the position where the cover stays on the chamber is random, and the position where the carrier 30 is located in the chamber is also random. Therefore, in some cases, the test strip is inserted into the groove of the carrier, and a part of the test strip is blocked by the label 210 at the bottom of the chamber (when there are many testing areas). In some cases, the carrier is just located in the window area reserved by the label, such that most of the grooves can be observed through the whole window area, and of course, most of the test strips can be seen, and actually one or more testing areas of the test strip can be seen. Therefore, when the chamber is inclined, whether any liquid has contacted with each testing area can be observed through the whole transparent area, then the chamber is inclined to stand again to wait for reaction, and the test results of the testing area are observed. This is the randomness of the actual screw thread and the conventional design method. If the testing area is blocked by the label, the color change of the testing area cannot be effectively observed.

A third way is that an integral protrusion structure is arranged on the edge of the opening of the chamber and can prevent the cover from movement. An arrangement way is that after the cover rotates for several weeks, once the cover is in contact with the protrusion blocking the cover, the cover cannot rotate and stays at the same position and the carrier 30 also stays at the same position. Reference to a way described in the patent announcement number US7300633B2 is made for this arrangement way. For example, a reference numeral 112 in this publication has a similar function and is considered as a barrier for the cover to seal the chamber in this US patent, and this structure is adopted in the invention to specifically position the cover. Therefore, the cover 10 drives the carrier 30 to be rotated into the window area, where the cover is blocked by the protrusion structure and can no longer be rotated. When testing needs to be performed, the liquid is directly inclined, and whether the liquid sample has contacted with the testing areas 51-54 is observed through the transparent area and whether each testing area contacts with the liquid sample. After a short contact, for example, 1-10 seconds, the chamber is immediately allowed to stand. In this case, the level of the liquid is located below the testing area 54 closest to the liquid in the test strip. After the reaction in the testing area, the color of the testing area is compared with that of the standard color card, and the results are positive or negative.

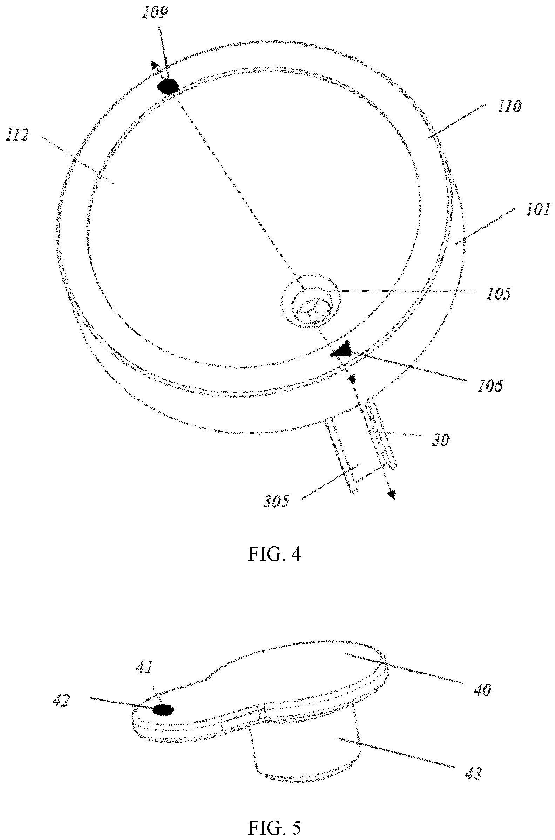

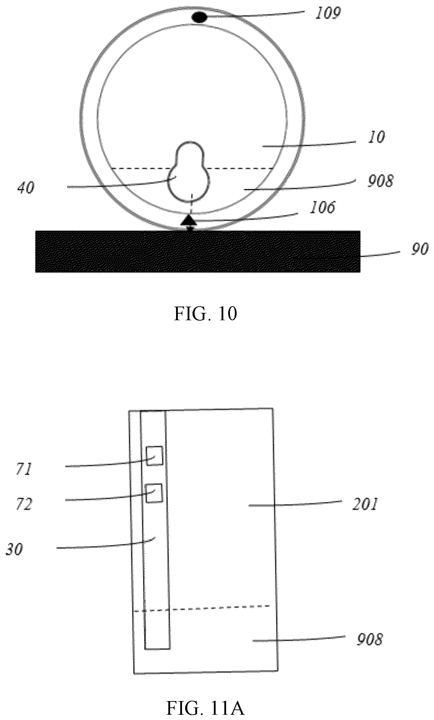

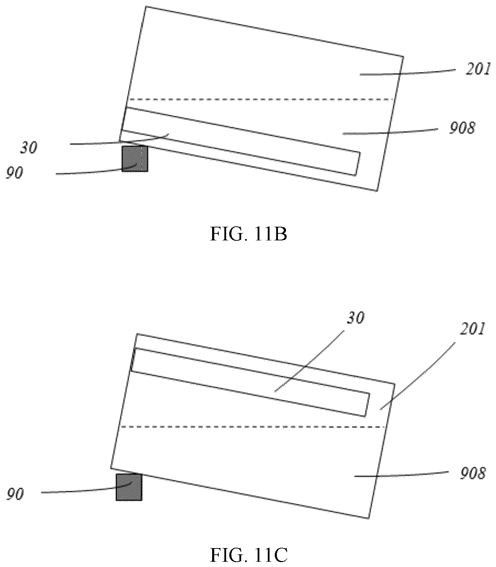

In some embodiments, no matter where the carrier 30 stays in the chamber 201, the liquid can be conveniently released from each testing area of the test strip through a simple operation. In this case, no matter where the carrier 30 stays in the chamber 201, the test strip is located in the groove of the carrier. This way is that a label is provided, and the label indicates that the chamber is inclined toward a direction in which a connection line between the the chamber and the hole 105 in the cover is shortest, and the liquid can be allowed to contact with all testing areas of the test strip. In one embodiment, one sealing plug 43 is further provided and seals the hole 105 in the cover 10 to prevent the liquid from leaking out of different holes 105 when the chamber 201 is inclined. In addition, if the test strip is relatively long and the part of the test strip is located in the hole 105, the sealing plug 43 can be used to fix the test strip when sealing the hole 105. In one embodiment, the sealing plug 43 has a sealing cover 40 that has a sealing head 41, and the sealing head has the function of direction indication, and the connection line between the sealing head and the label 106 in the cover may be shortest (the position of the sealing head is substantially the position of the hole 105 in the cover). In another embodiment, the cover 10 includes an indication label 106, the direction indicated by the indication label is the same as the direction of the opening of the groove of the carrier 30, and both the indication label and the opening face towards the transparent side walls of the chamber 201, as shown in dotted lines indicated by two arrows in FIG. 4. The indication label is located near the hole 105 in the cover and is a position label with a shortest distance to the hole; the carrier is below the label 106; and the indication label 106 is used for indicating the inclination direction of the chamber. When the sealing plug 43 seals the hole 105, the sealing head 41 of the sealing plug is aligned with the label 106 on the cover, and the sealing head 41 also has the function of direction indication and is used to indicate the inclination direction of the chamber. The "inclination direction" defined by the invention means that when the cover 10 covers the opening of the chamber 201, generally the testing areas 71, 72 of the test strip do not contact with the liquid sample 908 in the chamber. In this case, the chamber 201 contains the liquid sample 908 in a standing way (as shown in FIG. 11A), and the whole chamber 201 is inclined to allow the liquid to flow into the cover or the opening of the chamber (as shown in FIG. 11B) from the bottom 205 of the chamber. Therefore, the liquid sample 908 is laterally distributed in the chamber 201, and it is desired that the laterally distributed liquid sample contacts with the test strip on the laterally distributed carrier (after the chamber 201 is inclined, the carrier 30 is inclined and the test strip on the carrier will also be inclined), and specifically contacts with the testing area of the test strip. After a fixed time of contact, the chamber is allowed to immediately stand again and return to the position as shown in FIG. 11A. Thus, the direction indicated by the sealing head 41 is on a substantial plane with the carrier. Alternatively, there are several labels 42 on the sealing head, and the labels are aligned with the labels 106 on the cover. During specific operation, the liquid sample is collected in the chamber 201, and then the screw thread of the cover 10 is meshed with the screw thread at the opening of the chamber 201 for sealing. In this case, no matter where the carrier 30 is located, the test strip is inserted into the chamber 201 through the hole 105. If the liquid sample is within the maximum liquid level, only the non-absorbent support sheet of the test strip may contact with the liquid, but the test strip does not contact with the liquid sample. Then, the sealing plug 43 is used to seal the opening 105, and the sealing head 41 or the label 42 is aligned with the label 106 on the cover. When operation needs to be performed, the chamber is directly inclined, to be specific, the position 106 of the chamber with the label is inclined downward (for example, the position is inclined onto a table top 90); in this case, the liquid sample 908 located at the bottom of the chamber 201 is gathered in the corresponding chamber with the label 106, and the liquid contacts with the test strip or the testing area of the test strip, or the whole test strip will be immersed in the liquid (as shown in FIG. 10). For example, during the operation, the chamber is inclined for 1-10 seconds, and then the chamber is allowed to stand (return to the position as shown in FIG. 9) In this case, the test strip is still located in the groove, and the test results of the testing area can be read through the transparent side walls of the chamber. When the labeling way is adopted, no matter where the carrier 30 is located, to be specific, no matter where the test strip is vertically located in the chamber 201, during operation, the chamber is inclined towards a direction of a shortest connection line among the label 106, the piston head 41, the label 42 on the piston head and the piston hole; the test strip on the carrier can be immersed in the liquid sample, such that the operation is more convenient; regardless of the position of the cover, the position of the carrier, or the specific position of the test strip in the chamber, testing can be completed. After all, the carrier 30 is fixedly connected to the cover, and the test strip is inserted into the groove of the carrier through the hole 105 and fixed in the groove.

On the contrary, if there is no label, or if the label is inclined in a direction opposite to the position of the carrier, or if the label is not inclined in the direction of the shortest position from the hole 105, but inclined in other positions. Although the liquid samples are laterally distributed, they may not contact with the carrier; for example, the chamber is inclined towards the direction of the position 109, 1091 or 1092 as shown in FIG. 4. The most extreme direction is that the direction indicated by the farthest distance 109 from the hole is inclined, such that the liquid will not contact with the carrier (as shown in FIG. 11C), let alone the test strip on the carrier; and of course, the liquid will not contact with the testing area of the test strip, and testing will not be completed.

In some embodiments, as shown in FIG. 9, two sets of testing areas for testing the same analyte are provided on the test strip 60; one set of testing areas 61, 62 is provided at one end of the test strip while the other of testing areas 63, 64 is provided at the other end of the test strip; no matter which section of the test strip is inserted into the hole 105 of the cover and located in the groove of the carrier, testing can be completed. For example, as shown in FIG. 9, the testing area 64 is located in the liquid while the testing areas 61, 62, 63 are located above the liquid level instead of contacting the liquid sample. If the test strip as shown in FIG. 8 is used and oppositely inserted, and the test strip in a direction indicated by three arrows is inserted into the air, the testing area 71 in the testing areas 71, 72 near the arrows may always be in the liquid sample, and testing will not be completed.

### Embodiments

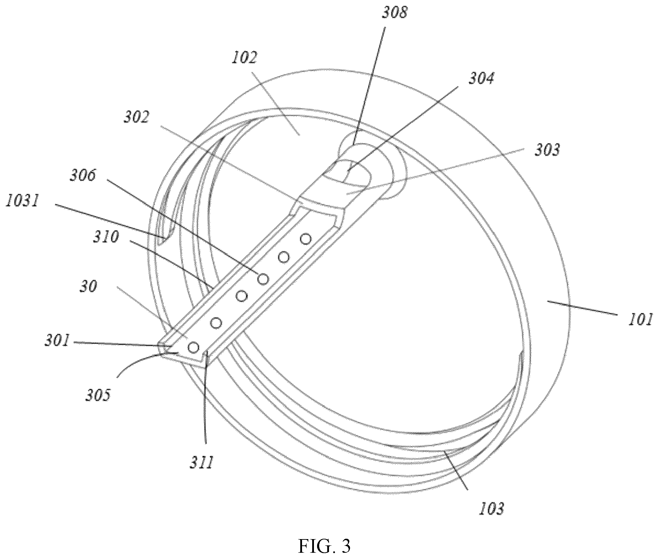

As shown in FIG. 1 - FIG. 6, and FIG. 8, the invention provides a testing device, and the testing device includes a chamber 201, where the chamber is configured to receive a liquid sample. The label 210 covers the bottom of the chamber, and the label has a maximum liquid level label 208 and a minimum liquid level label 209. The testing device further includes one cover 10, where the cover is provided with one carrier 30 with the groove and configured to allow the test strip 70 to lean against the groove 301 of the carrier, the carrier includes one hole 105, and the test strip can be inserted into the groove of the carrier through the hole 105. Moreover, there is the tubular structure 302 in the groove of the carrier near the cover, and a part of the test strip is located in the tubular structure, such that the test strip can be fixed in the groove without falling off. In some embodiments, one or more holes 306 are provided in the bottom surface 305 of the groove, and the holes are configured to allow a liquid to pass through and flow into the groove so as to contact with the test strip, especially for contacting with the testing areas of the test strip. In some embodiments, the carrier is located on the edge 110 of the cover, and one label 106 is provided at the position of the cover with the shortest distance from the hole, and this label has an arrow direction in which the chamber is inclined during operation (as shown in FIG. 4). One test strip 70 is provided as shown in FIG. 8 is made of a non-absorbent plastic 74 as a support sheet, and has two testing areas 71, 72, one testing area 71 is used to test white blood cells for testing urine samples, and the other testing area 72 is used to determine nitrous acid and located at one end of the test strip; there are three arrows 73 on the test strip, and the arrows means that the test strip is inserted into the hole 105 in the cover.

In operation, the chamber 201 is used to collect liquid, such as urine samples, until the amount of urine is above the maximum liquid level label 208 and can be located below the minimum liquid level label 209. Then, the chamber is allowed to stand. In this case, the cover 10 is used to cover the chamber, and the cover seals the chamber through thread engagement. Then, the sealing plug 40 is removed from the hole 105, and the test strip 70 is inserted into the hole 105 in the direction indicated by the arrow 73, such that the test strip 70 passes through the pipe 302 on the carrier and is located in the groove 305. In this case, the arrow of the test strip is close to the hole 105, and the testing areas 71, 72 are located in the groove below the pipe. Then, the hole 105 is plugged by the sealing plug. Then, the chamber is inclined along the direction indicated by the label 106 on the cover, as shown in FIG. 10, placed on the test bench for 5 seconds, and then the chamber is allowed to stand through the carrier. The color of the testing areas 71, 72 is observed and is compared with the color of the attached standard color card. In case of color change, the testing structure is judged according to the color of the standard color card. In absence of the color change of the testing areas, the test results are negative; in case of color change, the color of the testing areas is the same as the color of the standard color card, and the test results are positive. Results are judged to be valid if read within 3 minutes; and results are invalid if read for more than 3 minutes.

The following specific embodiments are also a part of the invention.

A device for testing an analyte in a liquid sample is provided and includes:
a chamber for receiving the liquid sample, where the chamber includes an opening and transparent side walls enclosing the chamber;
a cover, where the cover is configured to seal the opening of the chamber;
a carrier with a groove, where the carrier is configured to receive and hold a test strip and is provided with a tubular structure; and when the carrier is held in the groove, the tubular structure enables the test strip to be stably held in the groove instead of departing from the groove; and
the test strip, where the test strip includes one or more testing areas, and the testing areas are used for testing the presence or absence or quantity of the analyte in the liquid sample.

In some embodiments, the tubular structure includes a cover plate, and the cover plate covers a part of the groove.

In some embodiments, the groove is enclosed by a bottom surface and two side surfaces, the groove is provided with the opening, the opening faces side walls of a collection chamber, and the cover plate covers the opening of the part of the groove.

In some embodiments, the cover includes a hole, and the hole is connected to the carrier, such that the test strip is capable to be inserted into the groove of the carrier through the hole; and in an insertion process, the test strip passes through the tubular structure.

In some embodiments, the cover is provided with a label and a connection line between the label and the hole is shortest; and when testing is performed, the label is configured to indicate an inclination direction, such that the liquid sample contacts with the testing areas of the test strip in the groove.

In some embodiments, when testing is performed, the hole is configured to indicate an inclination direction, such that the liquid sample contacts with the testing areas of the test strip in the groove.

In some embodiments, the groove includes one or more holes, and the holes are configured to allow a liquid to pass through and flow into the groove so as to contact with the testing areas of the test strip.

In some embodiments, the carrier is fixedly connected to the cover.

In some embodiments, the tubular structure is adjacent to the cover.

In some embodiments, the carrier is opaque.

In some embodiments, an opaque label covers a bottom of the chamber, and carries a maximum liquid sample size label and a minimum liquid sample size label.

In some embodiments, the testing areas of the test strip include chemical substances, and the chemical substances are capable to react directly or indirectly with the analyte in the liquid sample to produce colored substances.

In some embodiments, the test strip is provided with two same testing areas that include same chemical substances and are used for testing same analytes, and the testing areas are respectively distributed at both ends of the test strip.

In some embodiments, the liquid sample is urine, the test strip includes a non-absorbent support sheet, the testing areas are located on the support sheet, and the testing areas include an absorbent material.

A method for testing an analyte in a liquid sample is provided and includes: providing a testing device, where the testing device includes: a chamber for receiving the liquid sample, where the chamber includes an opening and transparent side walls enclosing the chamber; a cover, where the cover is configured to seal the opening of the chamber; a carrier with a groove, where the carrier is configured to receive and hold a test strip and is provided with a tubular structure; and when the carrier is held in the groove, the tubular structure enables the test strip to be stably held in the groove instead of departing from the groove; the test strip, where the test strip is provided to test the analyte in the liquid sample, and includes a non-absorbent support sheet, testing areas are located on the support sheet, and the testing areas include an absorbent material;
the test strip is provided in the groove of the carrier, and a part of the test strip is located in the tubular structure; in this case, the testing areas are not in contact with the liquid sample, then the liquid sample is allowed to be in contact with the testing areas for a specified period of time, and finally the liquid sample is allowed to depart from the testing areas.

In some embodiments, the test strip is inserted into the groove of the carrier; and in an insertion process, the test strip passes through the tubular structure.

In some embodiments, the cover is fixedly connected to the carrier and has a hole; and in a testing process, the test strip is allowed to pass through the hole and to be located in the groove of the carrier.

In some embodiments, a way to allow the liquid sample to contact with the testing areas of the test strip comprises a way to allow the chamber to be inclined along a direction indicated by the hole in the cover, such that the liquid sample contacts with the testing areas for 1-20 seconds.

In some embodiments, after the liquid sample is allowed to contact with the testing areas, the chamber is allowed to stand again, such that the liquid sample departs from the testing areas.

In some embodiments, when the test strip is inserted into the groove of the carrier, the testing areas of the test strip are adjacent to the cover.

All the patents and publications mentioned in the description of the invention indicate that these are public technologies in the art and can be used by the invention. All the patents and publications cited herein are listed in the references, just as each publication is specifically referenced separately. The invention described herein can be realized in the absence of any one element or multiple elements, one restriction or multiple restrictions, where such restriction is not specifically described here. For example, the terms "comprising", "essentially consisting of" and "consisting of" in each example herein may be replaced by the rest 2 terms. The terms and expressions which have been employed herein are descriptive rather than restrictive, and there is no intention to suggest that these terms and expressions in this description exclude any equivalents, but it is to be understood that any appropriate changes or modifications can be made within the scope of the invention and appended claims.

## Claims

1. A device for testing an analyte in a liquid sample, comprising:
a chamber for receiving the liquid sample, wherein the chamber comprises an opening and transparent side walls enclosing the chamber;
a cover, wherein the cover is configured to seal the opening of the chamber;
a carrier with a groove, wherein the carrier is configured to receive and hold a test strip and is provided with a tubular structure; and when the test strip is held in the groove, the tubular structure enables the test strip to be stably held in the groove instead of departing from the groove; and
wherein the test strip comprises one or more testing areas, and the testing areas are used for testing the presence or absence or quantity of an analyte in the liquid sample.

2. The device according to claim 1, wherein the tubular structure comprises a cover plate, and the cover plate covers a part of the groove.

3. The device according to claim 2, wherein the groove is enclosed by a bottom surface and two sides, and has an opening; the opening faces side walls of a collection chamber; and the cover plate covers the opening of the part of the groove.

4. The device according to one any claims 1-3, wherein the cover comprises a hole, and the hole is connected to the carrier, such that the test strip is capable to be inserted into the groove of the carrier through the hole; and in an insertion process, the test strip passes through the tubular structure.

5. The device according to one any claims 1-4, wherein the cover is provided with a label and a connection line between the label and the hole is shortest; and when testing is performed, the label is configured to indicate an inclination direction, such that the liquid sample contacts with the testing areas of the test strip in the groove.

6. The device according to one of any claims 1-4, wherein when testing is performed, the hole is configured to indicate an inclination direction, such that the liquid sample contacts with the testing areas of the test strip in the groove.

7. The device according to one of any claims 1-6, wherein the groove comprises one or more holes, and the holes are configured to allow a liquid to pass through and flow into the groove so as to contact with the testing areas of the test strip.

8. The device according to one of any claims 1-7, wherein the carrier is fixedly connected to the cover.

9. The device according to one of any claims 1-8, wherein an opaque label covers a bottom of the chamber, and carries a maximum liquid level label and a minimum liquid level label.

10. The device according to one of any claims 1-9, wherein the testing areas of the test strip comprise chemical substances, and the chemical substances are capable to react directly or indirectly with the analyte in the liquid sample to produce colored substances.

11. The device according to one of any claims 1-10, wherein the test strip comprises a non-absorbent support sheet, the testing areas are located on the support sheet, and the testing areas comprise an absorbent material.

12. A method for testing an analyte in a liquid sample, comprising:
providing a testing device, wherein the testing device comprises:
a chamber for receiving the liquid sample, wherein the chamber comprises an opening and transparent side walls enclosing the chamber;
a cover, wherein the cover is configured to seal the opening of the chamber;
a test strip, wherein the test strip is provided to test the analyte in the liquid sample, and comprises a non-absorbent support sheet, testing areas are located on the support sheet, and the testing areas comprise an absorbent material;
the test strip is provided in the chamber, and in this case, the testing areas are not in contact with the liquid sample, then the liquid sample is allowed to be in contact with the testing areas for a specified period of time, and finally the liquid sample is allowed to depart from the testing areas.

13. The method according to claim 12, wherein a way to allow a liquid sample to contact with the testing areas comprises a way to allow the chamber to be inclined towards the test strip or to be inverted.

14. The method according to one of any claims 12-13, wherein a carrier with a groove is provided in the chamber, and the carrier is configured to receive and hold the test strip and is provided with a tubular structure; and when the carrier is held in the groove, the tubular structure enables the test strip to be stably held in the groove instead of departing from the groove.

15. The method according to claim 14, wherein the test strip is inserted into the groove of the carrier; and in an insertion process, the test strip passes through the tubular structure.

16. The method according to one of any claims 12-15, wherein the cover is fixedly connected to the carrier and has a hole; and in a testing process, the test strip is allowed to pass through the hole and to be located in the groove of the carrier.

17. The method according to claim 16, wherein a way to allow the liquid sample to contact with the testing areas of the test strip comprises a way to allow the chamber to be inclined along a direction indicated by the hole in the cover, such that the liquid sample contacts with the testing areas for 1-20 seconds.

18. The method according to one of any claims 12-17, wherein after the liquid sample is allowed to contact with the testing areas, the chamber is allowed to stand again, such that the liquid sample departs from the testing areas.
